Europäisches Patentamt

European Patent Office

Office européen des brevets

(19)

(11) Veröffentlichungsnummer: **0 503 397 A1**

## (12) EUROPÄISCHE PATENTANMELDUNG

(21) Anmeldenummer: **92103437.7**

(22) Anmeldetag: **28.02.92**

(51) Int. Cl.5: **A61B 1/26**

(30) Priorität: **08.03.91 CH 703/91**

(43) Veröffentlichungstag der Anmeldung:
**16.09.92 Patentblatt 92/38**

(84) Benannte Vertragsstaaten:
**DE FR GB NL SE**

(71) Anmelder: **Narco-Med AG**
**Hafenstrasse 20**
**CH-8280 Kreuzlingen(CH)**

(72) Erfinder: **Waldvogel, Herman Hans, Dr. med.**
**Allaman**
**F-74500 Lugrin(FR)**

(74) Vertreter: **Keller, René, Dr. et al**
**Patentanwälte Dr. René Keller & Partner**
**Postfach 12**
**CH-3000 Bern 7(CH)**

(54) **Laryngoskop.**

(57) Ein Laryngoskop (1) ist mit einer Kraftmeßeinrichtung ausgerüstet, um den zeitlichen Kraftaufwand eines Anästhesisten während des Freilegens und Freihaltens eines Weges im Mund- und Rachenraum zum Einführen eines Tubus in die Trachea messen und aufzeichnen zu können. Die Kraftmeßelemente (19, 29, 43) der Kraftmeßeinrichtung sind im Handgriff (8) des Laryngoskops (1) angeordnet und beeinträchtigen somit nicht die Sterilisationmöglichkeiten des Spatels (3).

Durch die Messung des zeitlichen Kraftaufwandes läßt sich einerseits objektiv die Fertigkeit des Anästhesisten beurteilen, andererseits können die für den Intubationsvorgang eingesetzten Pharmaka bezüglich ihrer pharmakologischen Eigenschaften, wie z. B. Hervorrufen einer schnellen und tiefen Muskelrelaxation, objektiv mit Meßwerten belegt werden.

Fig. 3

EP 0 503 397 A1

Die Erfindung betrifft ein Laryngoskopgemäß dem Oberbegriff des Patentanspruchs 1.

Laryngoskope werden insbesondere beim Menschen zum Niederdrücken des Zungengrundes während der Einführung eines Tubus vorbei an den Stimmritzen in die Trachea (Luftröhre) verwendet. Für die Intubation ist eine Lokalanästhesie oder Narkose mit oder ohne Muskelrelaxans notwendig, um eine Hyporeflexie und Muskelrelaxation des Pharynx- und Larynxmuskels zu erzielen.

Ein Laryngoskop dieser Art ist aus der US-PS 4,384,570 bekannt. Das bekannte Laryngoskop hat einen Handgriff, einen abgewinkelt in einen Spatelfuß übergehenden, seitensymmetrisch aufgebauten Spatel, der mit seinem Spatelfuß derart gegenüber dem Handgriff verdrehbar ist, daß der Winkel zwischen der Handgriffachse und der Symmetrieebene des Spatels einstellbar ist. An der im klinischen Einsatz den oberen Schneidezähnen des Patienten zugewandten Oberseite des Spatels ist eine Druckmeßplatte angeordnet. Die Druckmeßplatte ist mit einer Sicherheitseinrichtung elektrisch verbunden, die ein akustisches oder optisches Signal aussendet, falls ein übermäßiger Anpreßdruck auf die oberen Schneidezähne erfolgt.

Bislang ist es (auch mit dem gemäß US-PS 4,384,570 bekannten Laryngoskop) nicht möglich, objektiv den Intubationsvorgang zu erfassen: Es werden deskriptive Kriterien (z. B. schwierige Intubation - leichte Intubation) sowohl für das Anästhesieprotokoll als auch in der klinischen Erforschung für z. B. neue Muskelrelaxans gebraucht. Aus diesem Grunde ist es für die pharmazeutische Industrie äußerst schwierig in Hinblick auf die unterschiedlichen anatomischen Gegebenheiten der einzelnen Patienten, den unterschiedlichen Handfertigkeiten der ausführenden Anästhesisten und den oben genannten subjektiven Kriterien pharmazeutische Mittel für Lokalanästhetica, Muskelrelaxans und Anästhetika hinsichtlich ihrer pharmakologischen Eigenschaften und klinischen Anwendbarkeit auf ihre Einsatzfähigkeit zu überprüfen.

Der Erfindung liegt die Aufgabe zugrunde, ein Laryngoskop zu schaffen, mit dem objektive Kriterien und Merkmale einer optimalen Intubation ermittelbar sind, um einerseits die Ausbildung zukünftiger Anästhesisten zu vervollkommnen und andererseits objektiv die Wirkung von Lokalanästhetica, Muskelrelaxans und Anästhetika ermitteln zu können. Ein derartiges Laryngoskop sollte sich für die klinischen Anwendungen leicht sterilisieren lassen.

Durch die Erfindung, wie sie in den Patentansprüchen gekennzeichnet ist, wird die obige Aufgabe mit Hilfe eines Laryngoskops gelöst, mit welchem der Kraftaufwand zum Freilegen und Freihalten eines Weges für einen in die Trachea einzuführenden Tubus meßbar ist.

In den Patentansprüchen 2 bis 10 sind bevorzugte Ausführungsarten des erfindungsgemäßen Laryngoskops beschrieben.

Im folgenden werden Beispiele des erfindungsgemäßen Laryngoskops anhand von Zeichnungen näher erläutert. Es zeigen:

Fig. 1    eine Seitenansicht eines erfindungsgemäßen Laryngoskops,

Fig. 2    den Einsatz des Laryngoskops,

Fig. 3    eine Variante des in **Figur 1** dargestellten Laryngoskops, und

Fig. 4    eine weitere Variante des Laryngoskops.

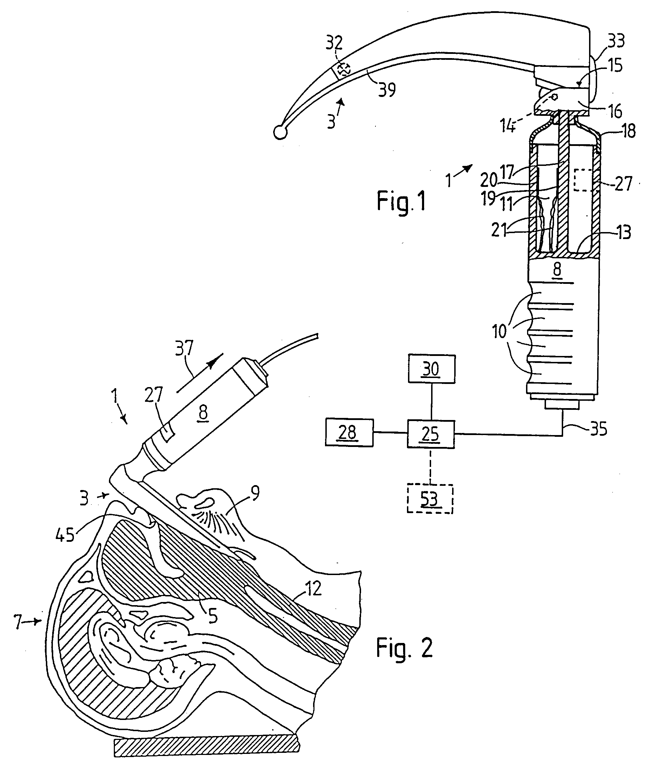

Der aufgeklappte Spatel **3** des in **Figur 1** dargestellten Laryngoskops **1** ist in den in **Figur 2** dargestellten Mund- und Rachenraum **5** eines Patienten **7** eingeführt. Durch Zug am Handgriff **8** des Laryngoskops **1**, wobei seine vier Finger in den in **Figur 1** dargestellten Fingerrillen **10** liegen, drückt der Anästhesist den Zungengrund **9** nieder, um den Weg für einen (nicht dargestellten) Tubus an den Stimmritzen vorbei in die Trachea **12** freizulegen und während dessen Einführens freizuhalten.

Der Spatel **3** ist mit einem Gelenk **14** als Verbindungsteil oben am Handgriff **8** befestigt und in **Figur 1** annähernd rechtwinkelig zu diesem aufgeklappt, wobei die mit dem Spatel **3** verbundene Gelenkauslenkungsanschlagfläche **15** auf der Auslenkungsanschlagfläche des Gelenkkopfes **16** an der der Spatelspitze abgewandten Seite aufliegt. Das Gelenk **14** ist derart ausgebildet, daß der Spatel **3** vom Handgriff **8** zur Sterilisation abnehmbar ist. Der Gelenkkopf **16** sitzt auf einem elastisch verbiegbaren Stab **17** als Spatelträger. Der Stab **17** ist in einem Hohlraum **11** im oberen Teil des Handgriffs **8** angeordnet und ist über den Hohlraumboden **13** starr mit dem Handgriff **8** verbunden. Die Stabachse verläuft annähernd parallel zur Achse des Handgriffs **8**. Die Federkraft des Stabes **17** ist so gewählt, daß er **17** auch bei unten beschriebenen größten Kraftanwendungen auf den Zungengrund **9** des Patienten nicht an den obersten Rand des Hohlraumes **11** anstößt. Ein offener Zwischenraum zwischen dem obersten Rand des Hohlraums **11** und dem Gelenkkopf **16** ist durch eine übergestülpte elastische Manschette **18** verschlossen.

Annähernd in der Mitte seiner Länge ist ein Dehnungsmeßstreifen **19** als Kraftmeßelement auf dem Mantel des Stabes **17** angeordnet. Die Senkrechte auf der Oberfläche des Dehnungsmeßstreifen **19** in Bereich dessen Längsmittellinie liegt senkrecht zur Achse des Gelenks **14** und annähernd parallel zur Spatellänge. Ein weiterer, zum Dehnungsmeßstreifen **19** analog ausgebildeter Dehnungsmeßstreifen **20** ist an der Innenwandung des Hohlraums **11** etwa dem Dehnungsmeßstreifen **19** gegenüberliegend angeordnet. Der Handgriff **8** und das Halteelement **17** sind aus gleichem Material hergestellt. Der Dehnungsmeßstreifen **20** dient als Kontrollelement zur Elimination thermischer Materialausdehnung. Die elektrischen Anschlußdrähte **21** der Dehnungsmeßstreifen **19** und **20** führen zu einer Auswerteeinrichtung **25**, die mit einer Anzeigeeinrichtung **25** im Handgriff **8**, einer weiteren ortsveränderlichen Anzeigeeinrichtung **27** und einem Schreiber **30** als Aufzeichnungsvorrichtung für die zeitabhängige Aufzeichnung der durch die Auswerteeinrichtung **27** ausgewerteten Meßwerte der Dehnungsmeßstreifen **19** und **20** dient. Da die Dehnungsmeßstreifen **19** und **20** gleich ausgebildet sind, kann mit der Auswerteeinrichtung **27** eine Differenzwertbildung der Meßwerte durchgeführt werden, wodurch eine Meßwertverfälschung durch thermische Längenänderungen des Stabes **17** eliminiert wird. Die Stromversorgung für die Kraftmeßvorrichtung und für eine Leuchte **32** im vorderen Drittel des Spatels **3** zur Beleuchtung des Mund- und Rachenraumes **5** während der Intubation ist nicht dargestellt.

Die Leuchte **32** ist über ein schematisch in **Figur 1** dargestelltes, flexibles Kabel **33** mit einem im Handgriff **8** verlaufenden (nicht dargestellten) Kabel verbunden, welches am unteren Ende des Handgriffs **8** mit einem weiteren Kabel **35** zur Auswerteeinrichtung **25** geführt ist.

Anstelle des flexiblen Kabels **33** und der Leuchte **32** kann auch ein im Gelenk **14** unterbrochener nicht dargestellter Lichtleiter verwendet werden, der in der Spatelspitze endet. Im Gelenk **14** liegen bei aufgeklapptem Spatel **3** die beiden Flächen des hier unterbrochenen Lichtleiters zur Überkopplung des Lichtes aufeinander. Durch eine im Handgriff **8** angeordnete Leuchte erhält der Lichtleiter sein Licht.

Zur Durchführung einer Intubation wird der Spatel **3**, wie bereits oben beschrieben, in den Mund- und Rachenraum **5** gesteckt und vom Anästhesisten mittels des Handgriffs **8** gegen den Zungengrund **9** in Pfeilrichtung **37** in **Figur 2** gezogen, wobei die auf den Zungengrund **9** drückende Andruckfläche **39** des Spatels **3** nach oben aufgrund der Elastizität des Stabes **17** geringfügigst weggebogen wird. Durch das elastische Verbiegen des Stabes **17** wird der Dehnungsmeßstreifen **19** gedehnt. Diese Dehnung ist unter Berücksichtigung des Auflageortes der Andruckfläche **39** auf dem Zungengrund **9** ein Maß für die auf die Andruckfläche **39** wirkende Kraft, welche dem Kraftaufwand des Anästesisten entspricht. Aufgrund der Formgebung des Spatels **3** ist dafür gesorgt, daß der Auflageort auf dem Zungengrund **9** sich annähernd immer an der gleichen Stelle befindet.

Die Umrechnung des mit dem Dehnungsmeßstreifen **19** ermittelten Dehnungswertes des Stabes **17** in einen Kraftwert, kann mittels einer in der Auswerteeinrichtung **25** gespeichertem mathematischen Formel erfolgen.

Anstelle den Kraftwert mathematisch zu bestimmen, können die Werte auch empirisch unter Verwendung eines Modells eines menschlichen Mund- und Rachenraumes bestimmt werden und die mit den Versuchen ermittelten Umrechnungswerte als Tabelle oder empirische Formel in der Auswerteeinrichtung **25** abgespeichert werden.

Anstelle nur eines einzigen Dehnungsmeßstreifens **19** auf dem Mantel des Halteelements **17**, kann auch noch ein zweiter, nicht dargestellte Dehnungsmeßstreifen, dessen Längsrichtung radial, also senkrecht zur Längsrichtung des Dehnungsmeßstreifens **19** verläuft, verwendet werden. Auch dieser zweite Dehnungsmeßstreifen ist mit der Auswerteeinrichtung **25** verbunden. Die Umrechnung der elektrischen Signale in Kraftwerte erfolgt analog zum Verfahren mit dem Dehnungsmeßstreifen **19**. Mit diesem zweiten Dehnungsmeßstreifen kann ein Verdrehen des Spatels **3** festgestellt werden. Anstelle eines Dehnungsmeßstreifens **19** kann auch ein weiterer oder können zwei weitere auf gleicher Höhe auf dem Mantel des Halteelements **17** zusätzlich angeordnet werden, wobei ein weiterer Dehnungsmeßstreifen vorzugsweise unter einem Winkel von 90° zum Dehnungsmeßstreifen **19** und zwei weitere unter Winkeln von 120° angeordnet werden. Durch das Anbringen mehrerer räumlich gegeneinander versetzter Kraftmeßelemente läßt sich der Wert und die Richtung der mit dem Spatel **3** aufgebrachten Kraft ermitteln.

Zusätzlich zu den oben beschriebenen Kraftmeßelementen, kann noch ein Druckaufnehmer, insbesondere ein auf einem im oberen Teil des Hohlraumes **11** angeordneten Ansatz **41** sitzender piezoelektrischer Druckaufnehmer **43** verwendet werden. Der Druckaufnehmer **43** ist ebenfalls mit der Auswerteeinrichtung **25** elektrisch verbunden. Mit den Druckaufnehmer **43** läßt sich ein auf den Handgriff **8** wirkendes Drehmoment bestimmen. Aus dem Verhältnis der mit dem Dehnungsmeßstreifen **19** und dem Druckaufnehmer **43** gemessenen Kräften läßt sich ferner bestimmen, ob der Anästesist nur den Zungengrund **9** alleine über mit dem Spatel **3** heruntergedrückt hat oder ob er zur Krafterparnis über die oberen Schneidezähne **45** des Patienten "gehebelt" hat. Ein derartiges "Hebeln" ist bei der Intubation nicht erlaubt, da es die Schneidezähne **45** des Patienten gefährdet.

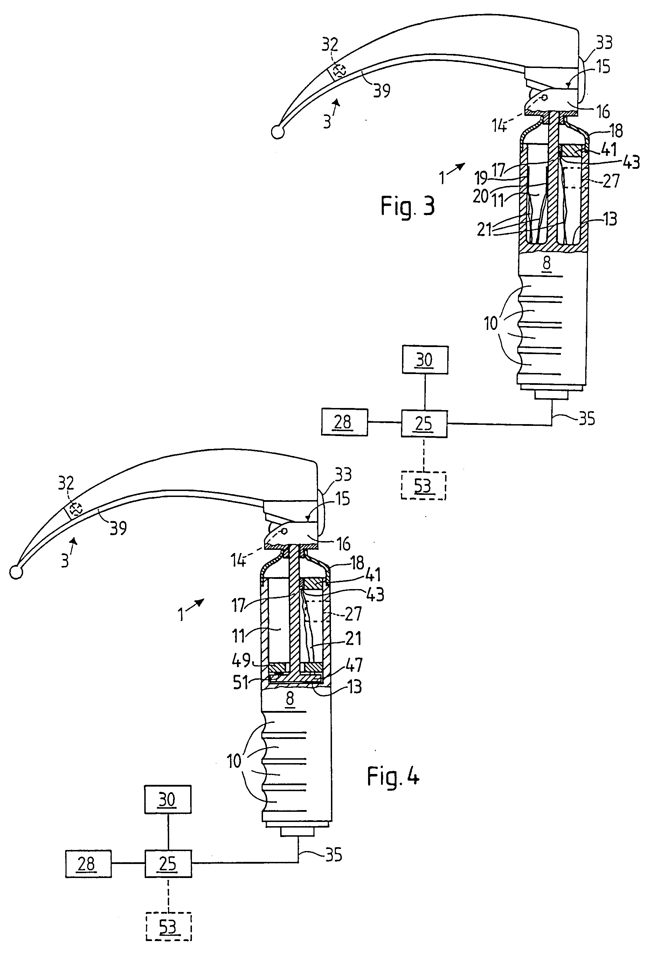

Anstelle der Dehnungsmeßstreifen **19** und **20** kann eine in **Figur 4** dargestellte Meßvariante verwendet werden. Hier ist das dem Gelenkkopf **16** abgewandte Ende des Spatelträgers **17** nicht mehr starr mit dem Hohlraumboden **13** verbunden, sondern trägt einen Teller **47**, der gegen einen ringförmigen Steg **49** oberhalb des Hohlraumbodens **13** drückt. Zwischen dem Steg **49** und der Telleroberseite ist dem Spatel **3** zugewandt ein mit der Auswerteeinrichtung **25** verbundener Drucksenor **51** z. B. ein piezoelektrischer Drucksenor angeordnet. Dieser Drucksensor **51** liefert zum Dehnungsmeßstreifen **19** analoge Ergebnisse. In dieser Konstruktionsvariante wird die elastische Verformung des Spatelträgers **17** nicht berücksichtigt.

Der Spatelträger kann auch als (nicht dargestellter) Kolben einer (nicht dargestellten) Kolbenzylindereinheit im Handgriff ausgebildet werden. Über eine mit dem Zylinder durch eine Druckleitung verbundene Anzeigeeinrichtung kann dann die vom Spatel auf den Kolben während der Intubation wirkende Zugkraft angezeigt werden. Eine derartige konstruktive Ausgestaltung hat den Vorteil, daß sie ohne elektrische Meßwertverarbeitung auskommen würde. In der Anzeigeeinrichtung ließe sich z. B. eine mechanische, wieder lösbare Arretierung für den maximalen Kraftwert anbringen.

Der Spatelträger könnte auch durch eine Feder, deren Auslenkung durch eine Anzeigeeinrichtung angezeigt würde, analog einer Federwaage gegen die bei der Intubation aufgewandten Kräfte im Handgriff abgestützt werden.

Die ermitteln zeitlichen Kraftverläufe lassen Rückschlüsse über die "handwerklichen" Fertigkeiten und damit über den Ausbildungsstand des Anästhesisten zu. Mehrere von Normwerten abweichende zu hohe und/oder zu lang andauernde Kraftaufwendungen lassen auf ungenügende Routine des Anästhesisten schließen.

Wird zusätzlich zur Zeit-Kraftkurve der Blutdruck des Patienten **7** aufgezeichnet, so kann bei einem stark ansteigendem Blutdruck im Bereich des größten Kraftaufwandes darauf geschlossen werden, daß die Narkosetiefe nicht ausreichend war.

Wird die Wirkung verschiedener Narkotika getestet, so ist der Verlauf der Zeit-Kraftkurve als objektives Vergleichsmittel zu verwenden.

Anstelle die Daten des zeitlichen Kraftaufwands über die separate Auswerteeinrichtung **25** auszuwerten und mit dem Schreiber **30** aufzuzeichnen, können die Daten auch mit einer im Handgriff **8** eingebauten, Auswerteeinrichtung **25** aufgearbeitet werden, welche einerseits den jeweils ermittelten Wert, wie oben beschrieben, mit der Anzeigeeinrichtung **27** im Handgriff **8** anzeigt und andererseits die zeitabhängigen Daten in einen im Handgriff **8** eingebauten (in den **Figuren 1**, **3** und **4** gestrichelt dargestellten) Speicher **53** ablegt, der über ein (nicht dargestelltes) Interface im Handgriff **8** mit einem externen (nicht dargestellten) Lesegerät verbindbar ist. Zur Stromversorgung der elektronischen Baugruppen (Speicher **53**, Auswerteeinrichtung **25**, Anzeigeeinrichtung **27**) ist im Handgriff **8** eine (nicht dargestellte) Batterie eingebaut.

Die im Speicher **43** abgelegten Daten können über das Interface auch direkt von einem (nicht dargestellten) Computer abgerufen und anschließend verarbeitet werden, um z. B. gemittelte Werte, "individuelle Diagramme" der verschiedenen Anästhesisten, statistische Streuungen, etc. zu ermitteln.

Als Aufzeichnungsvorrichtungen können neben analogen und digital arbeitenden Schreibern **30** auch Bildschirme verwendet werden.

Durch das erfindungsgemäße Laryngoskop ist es erstmals möglich, den zeitlichen Kraftaufwand zum Freilegen und Freihalten des Weges für einen in die Trachea einzuführenden Tubus objektiv zu messen. Durch diese Messung läßt sich einerseits die Fertigkeit des Anästhesisten beurteilen, andererseits können objektiv die für den Intubationsvorgang eingesetzten Pharmaka bezüglich ihrer pharmakologischen Eigenschaften, wie z. B. Hervorrufen einer schnellen und tiefen Muskelrelaxation, mit Meßwerten belegt werden.

Durch den Einbau der Meßelemente im Handgriff **8** entfallen alle Sterilisationsprobleme, da der Spatel **3**, wie oben dargestellt vom Handgriff **8** abnehmbar ist.

## Patentansprüche

1. Laryngoskop **(1)** mit einem Spatel**(3)**, einem Handgriff **(8)**, der mittels eines Verbindungsteils **(14)** mit dem Spatel **(3)** verbunden ist, und einer Kraftmeßeinrichtung zur Messung von mit dem Spatel **(3)** ausgeübter Kräfte, **dadurch gekennzeichnet**, daß das Kraftmeßelement **(19, 20, 43, 51)** der Kraftmeßeinrichtung im oder am Handgriff **(8)** angeordnet und so ausgebildet ist, daß die beim Freilegen und Freihalten eines Weges für einen in die Trachea **(12)** einzuführenden Tubus durch den Spatel **(3)** auf den Zungengrund **(9)** ausgeübte Kraft meßbar ist.

2. Laryngoskop **(1)** nach Anspruch 1, **gekennzeichnet durch** eine an die Kraftmeßeinrichtung angeschlossene oder anschließbare Aufzeichnungseinrichtung **(25, 30)** zur Darstellung und/oder Speicherung des Kraftaufwands in Abhängigkeit von der Zeit.

3. Laryngoskop **(1)** nach Anspruch 1 oder 2, **gekennzeichnet durch** eine im Handgriff **(8)** angeordnete Kraftanzeige **(27)**.

4. Laryngoskop **(1)** nach einem der Ansprüche 1 bis 3, **dadurch gekennzeichnet**, daß am oder im Handgriff **(8)** ein am einen Ende mit dem Verbindungsteil **(14)** starr oder gelenkig mit dem Spatel **(3)** verbundener Spatelträger **(17)** gehalten ist, und das Meßelement **(19, 43, 51)** so angeordnet und ausgebildet ist, daß die vom Handgriff **(8)** über den Spatelträger **(17)**, den Verbindungsteil **(14)** und den Spatel **(3)** auf den Zungengrund **(9)** ausgeübte Kraft als auf den Spatelträger **(17)** wirkende Zug- und/oder Biegekraft meßbar ist.

5. Laryngoskop **(1)** nach Anspruch 4, **dadurch gekennzeich**net, daß das Meßelement **(19, 43, 51)** am Spatelträger **(17)** oder an einem Halteteil **(41, 47, 49)** für den Spatelträger **(17)** zum Handgriff **(8)** angeordnet ist.

6. Laryngoskop **(1)** nach Anspruch 4 oder 5, **dadurch gekennzeichnet**, daß die Kraftmeßeinrichtung ein zweites **(43)** vom Angriffspunkt des ersten Meßelements **(19, 51)** am Spatelträger **(17)** distanziert angeordnetes Meßelement **(43)** aufweist, um unterschiedliche vom Spatel **(3)** auf den Spatelträger **(17)** wirkende Kraftkomponenten zu bestimmen.

7. Laryngoskop **(1)** nach Anspruch 6, **dadurch gekennzeichnet**, daß das eine **(43)** der beiden Meßelemente **(19, 43; 51, 43)** zwischen dem Spatelträger **(17)** und einer am Handgriff **(8)** befestigten Stütze **(41)** als Halteteil zur Abstützung des Spatelträgers **(17)** angeordnet ist, um das vom Spatel **(3)** auf den Spatelträger **(17)** ausgeübte Drehmoment zu messen.

8. Laryngoskop **(1)** nach einem der Ansprüche 1 bis 5, **dadurch gekennzeichnet**, daß das Kraftmeßelement oder wenigstens eines der Kraftmeßelemente als Druckaufnehmer, insbesondere als piezoelektrischer Druckaufnehmer **(43, 51)**, ausgebildet ist.

9. Laryngoskop **(1)** nach Anspruch 4 oder 5, **dadurch gekennzeichnet**, daß der Spatelträger ein elastisch verformbarer Stab **(17)** ist, auf dessen Mantel mindestens ein Dehnungsmeßelement **(19)** als Meßelement angeordnet ist.

10. Laryngoskop nach einem der Ansprüche 1 bis 9, **dadurch gekennzeichnet**, daß der Spatel **(3)** abnehmbar ist.

Fig.1

Fig. 2

Fig. 3

Fig. 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| P,A | EP-A-0 417 038 (NARCO-MED AG)<br>* Spalte 2, Zeile 18 - Spalte 5, Zeile 8; Abbildungen 1-3 *<br>--- | 1,2,9,10 | A61B1/26 |
| X | MEDICAL & BIOLOGICAL ENGINEERING & COMPUTING Bd. 21, Nr. 4, Juli 1983, STEVENAGE,GB Seiten 525 - 527;<br>R.T.CHILCOAT ET AL: 'A measuring laryngoscope handle: a device for measuring the force applied during laryngoscopy'<br>* Seite 525, linke Spalte, Zeile 1 - Seite 527, rechte Spalte, Zeile 18; Abbildungen 1-3 *<br>----- | 1-10 | |
| | | | **RECHERCHIERTE SACHGEBIETE (Int. Cl.5)**<br><br>A61B |

Der vorliegende Recherchenbericht wurde für alle Patentansprüche erstellt

| Recherchenort | Abschlußdatum der Recherche | Prüfer |
|---|---|---|
| BERLIN | 12 MAI 1992 | WEIHS J. |